# EUROPEAN PATENT APPLICATION

(11) **EP 4 527 303 A1**
(43) Date of publication of application: **26.03.2025**
(21) Application number: 24200946.2
(22) Date of filing: 18.09.2024
(51) Int. Cl.: A61B 6/03, A61B 6/00, A61B 6/51

(54) **A SOLUTION FOR A DENTAL X-RAY IMAGING OF AN OBJECT**

(30) Priority: 20.09.2023 FI 20236042
(71) Applicant: PaloDEx Group Oy, 04301 Tuusula (FI)
(72) Inventor: POLLARI, Mika, 01520 VANTAA (FI)
(74) Representative: Berggren Oy

(57) **Abstract**

The invention relates to a dental X-ray imaging system (100) for a computed tomography (CT) dental X-ray imaging of an object (300). The system (100) comprises: a dental X-ray imaging unit (102) comprising: an X-ray source part (114), an X-ray imaging detector part (116), and a gantry part (112); and a control system (106). The control system (106) is configured to: receive scan request comprising an indication of at least one region of interest (ROI); control the parts of the dental X-ray imaging unit (102) to acquire two X-ray scout images (302, 304) of the object (300); detect at least one target structure of the object (300) from the two X-ray scout images (302, 304) associated with the at least one ROI by applying at least one trained detection model (616) for the X-ray scout images (302, 304); define field of view (FOV) data of the dental X-ray imaging unit (102) at least partially based on the detected at least one target structure; and control the parts of the dental X-ray imaging unit (102) to implement the defined FOV data for a CT scan the object (300) in order to acquire dental CT X-ray image data of the object (300). The invention relates also to a method for a computed tomography (CT) dental X-ray imaging, a computer program and a tangible nonvolatile computer-readable medium.

## Description

### TECHNICAL FIELD

The invention concerns in general the technical field of dental X-ray imaging. Especially the invention concerns computed tomography (CT) dental X-ray imaging.

### BACKGROUND

A dental X-ray imaging unit typically comprises a gantry part, i.e. a rotating part, having an X-ray source part and an X-ray imaging detector part. The X-ray source part provides an X-ray beam that travels through an object, e.g. a head of a patient, to the X-ray imaging detector part. In a computed tomography (CT) dental X-ray imaging, the X-ray beam forms a cylindrical field of view (FOV) around a vertical rotation axis, when the gantry part is arranged to rotate around the object. The position and the dimensions of the cylindrical FOV should be defined so that anatomical structures of the object to be included in the CT scan are inside the cylindrical FOV.

Patient supporting accessories are typically used to support the head of the patient as stationary as possible. The use of patient supporting accessories intended for certain specific regions (i.e. certain specific anatomical structures of the patient) of the object (e.g. front teeth and/or temporomandibular joints (TMJs)) enable that said certain specific regions are in a known position with respect the dental X-ray imaging unit and thus the 3D location of the FOV may be known a priori. However, this enables only that said specific regions may be imaged reliably, but there are not patient supporting accessories for other regions. Furthermore, the assembly of the patient supporting accessories for these certain specific regions is time consuming.

One traditional FOV positioning and dimensioning approach is based on using X-ray scout images, i.e. a set of two X-ray projection images taken typically at 90 degrees angle, wherein a user, e.g. an operator, of the dental X-ray imaging unit manually positions and dimensions the FOV by means of the X-ray scout images. The manual FOV positioning and dimensioning by the operator is time consuming and requires anatomy knowledge and skills by the operator.

Another known FOV positioning and dimensioning approach is based on using optical scout image, wherein the user of the dental X-ray imaging unit manually positions and dimensions the FOV by means of the optical scout image. However, the optical scout image -based FOV positioning and dimensioning is inaccurate, because the dental anatomical structures of the patient are not shown in the optical scout image. Thus, the X-ray scout image -based FOV positioning and dimensioning is also needed in addition to the optical scout image -based FOV positioning and dimensioning, which, in turn, increases the imaging time. Moreover, the optical scout image -based FOV positioning and dimensioning requires that the X-ray dental imaging unit comprises one or more optical cameras, which increases the price of the X-ray dental imaging unit.

### SUMMARY

The following presents a simplified summary in order to provide basic understanding of some aspects of various invention embodiments. The summary is not an extensive overview of the invention. It is neither intended to identify key or critical elements of the invention nor to delineate the scope of the invention. The following summary merely presents some concepts of the invention in a simplified form as a prelude to a more detailed description of exemplifying embodiments of the invention.

An objective of the invention is to present a dental X-ray imaging system and a method for a computed tomography (CT) dental X-ray imaging of an object. Another objective of the invention is that the dental X-ray imaging system and the method for the CT dental X-ray imaging of an object enables reducing imaging time and improving imaging accuracy.

The objectives of the invention are reached by a dental X-ray imaging system, a method, a computer program, and a tangible non-volatile computer-readable medium as defined by the respective independent claims.

According to a first aspect, a dental X-ray imaging system for a computed tomography (CT) dental X-ray imaging of an object is provided, wherein the system comprises: a dental X-ray imaging unit comprising: an X-ray source part for emitting X-rays, an X-ray imaging detector part for receiving the X-rays from the source part, and a gantry part comprising the X-ray source part and the X-ray imaging detector part, and a control system configured to: receive scan request comprising an indication of at least one region of interest (ROI); control the parts of the dental X-ray imaging unit to acquire two X-ray scout images of the object; detect at least one target structure of the object from the two X-ray scout images associated with the at least one ROI by applying at least one trained detection model for the X-ray scout images; define field of view (FOV) data of the dental X-ray imaging unit at least partially based on the detected at least one target structure; and control the parts of the dental X-ray imaging unit to implement the defined FOV data for a CT scan the object in order to acquire dental CT X-ray image data of the object.

The FOV data may comprise a FOV position and a FOV size.

The two X-ray scout images of the object may comprise a lateral (LAT) view angle X-ray scout image and a posterior-anterior (PA) view angle X-ray scout image.

The control system may further be configured to: obtain imaging geometry data representing an imaging geometry of the dental X-ray imaging unit respective to the two X-ray scout images; and transform the detected at least one target structure into 3D device coordinates by using the obtained imaging geometry data.

The scan request may further comprise object data representing at least one object related parameter and/or imaging program data representing required imaging program.

The object data and/or the imaging program data may be used in the defining of the FOV data.

The at least one trained detection model may be a machine learning (ML) model or an artificial intelligence (Al) model.

The at least one trained detection model may comprise at least one common trained detection model may be trained for both X-ray scout images.

Alternatively, the at least one trained detection model may comprise different at least one trained detection model may be trained for each X-ray scout image.

Alternatively or in addition, the at least trained detection model may depend on a region of the object imaged in the X-ray scout images.

The detecting the at least one target structure of the object from the two X-ray scout images may be based on landmark -based detection, bounding box - based detection, and/or object segmentation -based detection.

In the landmark -based detection at least one landmark associated with the at least one ROI may be detected from the two X-ray scout images by applying the at least one trained detection model for the X-ray scout images.

According to a second aspect, a method for a computed tomography (CT) dental X-ray imaging is provided, wherein the method is performed by an X-ray dental imaging system described above, wherein the method comprises: receiving scan request comprising an indication of at least one region of interest (ROI); controlling the parts of the dental X-ray imaging unit to acquire two X-ray scout images of the object; detecting at least one target structure of the object from the two scout images associated with the at least one ROI by applying at least one trained detection model for the X-ray scout images; defining field of view (FOV) data of the dental X-ray imaging unit at least partially based on the detected at least one target structure; and controlling the parts of the dental X-ray imaging unit to implement the defined FOV data for a CT scan of the object in order to acquire CT dental X-ray image data of the object.

According to a third aspect, a computer program is provided, wherein the computer program comprises instructions which, when the program is executed by an X-ray dental imaging system described above, cause the X-ray dental imaging system to carry out the method described above.

According to a fourth aspect, a tangible non-volatile computer-readable medium is provided, wherein the computer-readable medium comprises instructions which, when executed by an X-ray dental imaging system described above, cause the X-ray dental imaging system to carry out the method described above.

Various exemplifying and non-limiting embodiments of the invention both as to constructions and to methods of operation, together with additional objects and advantages thereof, will be best understood from the following description of specific exemplifying and non-limiting embodiments when read in connection with the accompanying drawings.

The verbs "to comprise" and "to include" are used in this document as open limitations that neither exclude nor require the existence of unrecited features.

The features recited in dependent claims are mutually freely combinable unless otherwise explicitly stated. Furthermore, it is to be understood that the use of "a" or "an", i.e. a singular form, throughout this document does not exclude a plurality.

### BRIEF DESCRIPTION OF FIGURES

The embodiments of the invention are illustrated by way of example, and not by way of limitation, in the figures of the accompanying drawings.
Figure 1 illustrates schematically an example of a dental X-ray imaging system for dental X-ray imaging of an object.
Figure 2 illustrates schematically an example of a method for CBCT dental imaging of an object.
Figure 3A illustrates schematically an example of lateral (LAT) imaging of an object.
Figure 3B illustrates schematically an example of a posterior-anterior (PA) imaging an object.
Figure 3C illustrates schematically an example of a LAT scout image of an object.
Figure 3D illustrates schematically an example of a PA scout image of an object.
Figure 4A illustrates schematically a non-liming example of landmarks intended for detecting maxillary teeth from a LAT scout image of an object.
Figure 4B illustrates schematically a non-liming example of landmarks intended for detecting maxillary teeth from a PA scout image of an object.
Figure 5A illustrates schematically an example of adjusting a horizontal dimension of a field-of-view (FOV).
Figure 5B illustrates schematically an example of adjusting a vertical dimension of the FOV.
Figure 6 illustrates schematically an example of a control system of a dental X-ray imaging system.

### DESCRIPTION OF THE EXEMPLIFYING EMBODIMENTS

In this description we use the following vocabulary concerning different phases of a dental X-ray imaging process. The term radiating means the phase comprising merely the irradiation, i.e. the phase when an X-ray source is providing an X-ray beam that travels through an object to an X-ray imaging detector. The object may be expected to remain as still, i.e. immobile, as possible during the radiating. During the radiating one or more parts of the dental X-ray imaging unit may move. The term scanning, in turn, means the phase comprising the radiating and moving of one or more parts of the dental X-ray imaging unit. The scanning does not comprise positioning of one or more parts of the X-ray imaging unit in a correct place for providing X-ray images. The term imaging means the whole process comprising radiating, scanning and positioning.

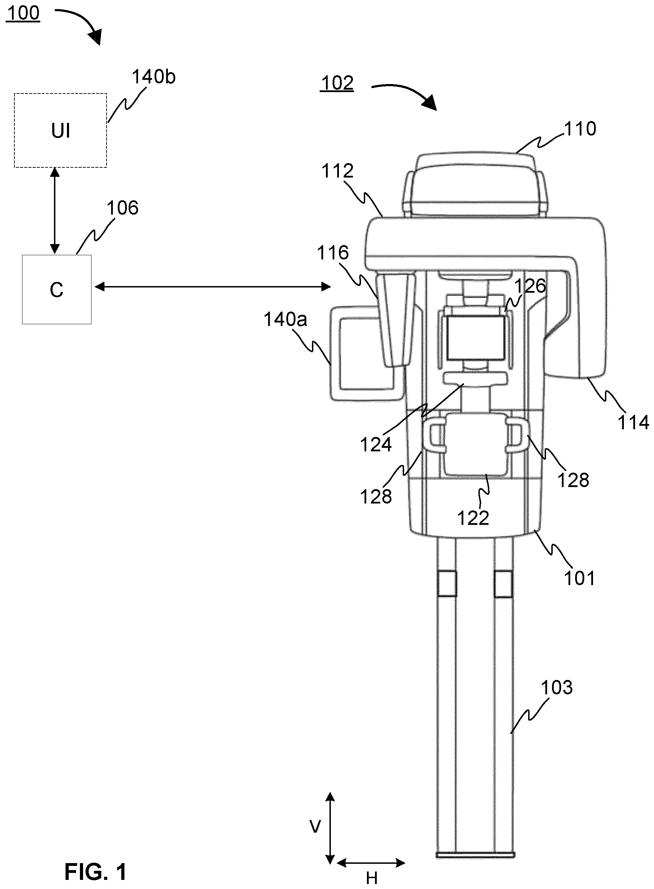

Figure 1 illustrates an example of a dental X-ray imaging system 100 for dental X-ray imaging of an object 300, e.g. a patient (for sake of clarity the object 300 is not shown in Figure 1). The imaging system 100 comprises a dental X-ray imaging unit 102 for acquiring X-ray image data from the object 300 or calibration target, in dental X-ray imaging, e.g. in extraoral dental X-ray imaging. The acquired X-ray image data is used to reconstruct a three-dimensional (3D) X-ray volume from at least part of the imaged object 300. The dental X-ray imaging system 100 further comprises a control system 106. The control system 106 may be electrically and/or communicatively coupled to the dental X-ray imaging unit 102. The implementation of the control system 106 may be done as a stand-alone unit or as a distributed control environment between a plurality of stand-alone units providing distributed controlling resource. Preferably, the control system 106 may be an embedded computer. The control system 106 may for example comprise a control unit of the dental X-ray imaging unit 102. The control system 106 may further comprise one or more computing units being external to the dental X-ray imaging unit 102. The control unit of the dental X-ray imaging unit 102 is configured to control the operation of the dental X-ray imaging unit 102 at least in part. The control unit of the dental X-ray imaging unit 102 may be located proximate to the dental X-ray imaging unit 102 or the control unit of the dental X-ray imaging unit 102 may be embedded withing the dental X-ray imaging unit 102.

The dental X-ray imaging unit 102 is configured to perform computed tomography (CT) imaging procedure (i.e. imaging mode). The CT imaging may for example be a cone beam CT (CBCT) imaging, wherein the beam is a cone-shaped beam, or other type of CT imaging for example, wherein the beam is a pyramidal-shaped beam, half-moon -shaped cone beam, or any other shaped beam. The CT imaging results (i.e. produces) the X-ray image data for the reconstruction of the 3D volume from the at least part of the imaged object 300. Figure 1 illustrates only one example of a dental X-ray imaging unit 102 for use with the concepts in the present disclosure.

The dental X-ray imaging unit 102 comprises a carriage part 101 that may be moveably supported on a support column 103. The carriage part 101 may be moved up and down in a vertical direction (V) by means of a guide motor (not shown in Figure 1) that is configured to move the carriage 101 up and down along the supporting column 103 in the vertical direction. An upper shelf, 110 is configured to support a gantry part, i.e. a rotating part, 112, which is rotatable in a horizontal (H) plane with respect to the upper shelf 110. The upper shelf 110 and/or the gantry part 112 may comprise a rotating motor (not shown in Figure 1) configured to rotate the gantry part 112. Alternatively or in addition, the upper shelf 110 and/or the gantry part 112 may comprise a pivot motor (not shown in Figure 1) configured to pivot the upper shelf 110 around the column 103. The upper shelf 110 and/or the gantry part 112 may alternatively or in addition comprise at least one linear motor (not shown in Figure 1) configured to provide linear movement(s). Alternatively or in addition, the dental X-ray imaging unit 102 may be mounted to a supporting structure (not shown in Figure 1) exemplarily a wall to being supported by the column 103.

The dental X-ray imaging unit 102 comprises further an X-ray source part 114 and an X-ray imaging detector part 116, which are used in the acquisition of the X-ray image data. The gantry part 112 embodies and supports the source part 114 and the imaging detector part 116. The gantry part 112 may have substantially a form of letter C, as presented in Figure 1, whereupon the source part 114 may be attached on one end of the gantry part 112 and the imaging detector part 116 may be attached on the other end of the gantry part 112 so that the source part 114 and the imaging detector part 116 are opposed from each other. The X-ray source part 114 comprises an X-ray source that emits X-rays (i.e. generates the X-ray beam) through the object 300 being imaged, e.g. a head of the patient, to the X-ray imaging detector part 116, which comprises at least one X-ray detector that receives the emitted X-rays from the source part 114. The X-ray imaging detector part 116 further generates the X-ray image data from the X-ray exposed, i.e. imaged object.

The dental X-ray imaging unit 102 also comprises a collimator 506 (not shown in Figure 1) for the X-ray source part 114 to restrict and/or shape the beam of X-rays. The X-rays pass through a portion of the object 300, for example the patient's anatomy, e.g. patient's head. The anatomical structures through which the X-rays pass may absorb varying amounts of the X-ray energy. After passing through the object 300, the attenuated X-rays are received by the X-ray imaging detector part 116. The X-ray imaging detector part 116 is configured to convert the magnitude of the received X-ray energy and to produce a digitized output, i.e. the X-ray image data, representative of the unabsorbed X-rays at the at least one X-ray detector. The collection of digitized outputs from the X-ray imaging detector part 116 that correspond to a single emission of a beam of X-rays from the X-ray source part 114 may be referred to a projection image of the object 300 being imaged, for example the head of the patient 300.

Furthermore, the dental X-ray imaging unit 102 may comprise patient support parts 124, 126 (as presented in Figure 1, but not necessarily) that may be used for supporting the patient. The patient support parts 124, 126 may comprise a chin support part 124 and/or a head support part 126. The chin support part 124 may support a tip of a chin of patient and the head support part 126 may support a forehead or temple of the patient. The dental X-ray imaging unit 102 may comprise a lower shelf 122 that extends from the carriage 101. The lower shelf 122 may comprise the chin support part 124 as in the example dental X-ray imaging unit 102 of Figure 1. The head support part 126 may extend from the upper shelf 110 through the gantry part 112 as in the example dental X-ray imaging unit 102 of Figure 1. Alternatively, the lower shelf 122 may also comprise the head support part 126. The patient support parts, i.e. the chin support part 124 and/or the head support part 126, may be optional, and positioning of the patient may be carried out in other manners. The dental X-ray imaging unit 102 may further comprise handles 128 for the patient to grasp.

The gantry part 112 may be rotated by a rotating motor, for example. The rotation of the gantry part 112 rotates the X-ray source part 114 and the X-ray imaging detector part 116 around the object to be imaged, for example around a rotation axis along a motion path. The X-ray beam emitted by the X-ray source part 114 forms a 3D cylindrical field-of-view (FOV) around the rotation axis, when the gantry part 112 rotates around the patient. The 3D cylindrical FOV should be dimensioned and positioned so that the anatomical structure(s) of the object 300 to be included in the scan is/are inside the 3D cylindrical FOV. As the X-ray source part 114 and the X-ray imaging detector part 116 are rotated around the object 300, the dental X-ray imaging unit 102 operates to acquire a plurality of projection images of the object 300 taken at incremental angles of rotation. The dental X-ray image may be formed from the plurality of projection images by reconstructing the X-ray image data to the dental X-ray image.

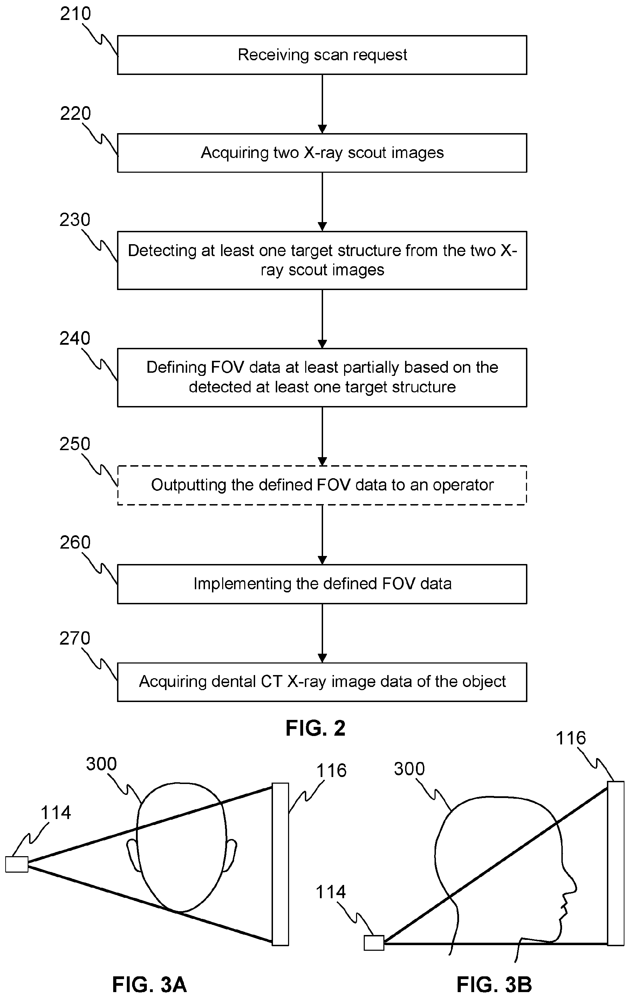

Next at least some example aspects of a method for CT dental X-ray imaging of an object 300, e.g. a patient, are defined referring to Figure 2. Figure 2 illustrates the method as a flow chart. The method is performed by the dental X-ray system 100 discussed above. The CT dental imaging of the object 300 may preferably be CBCT dental imaging.

At a step 210, the control system 106 receives a scan request. The scan request comprises an indication of at least one region of interest (ROI). The at least one ROI represents at least one region (e.g. at least one target anatomical structure) of the object 300 to be included in the CT scan. For example, the at least one ROI may comprise, but is not limited to, at least one of the following: a single tooth, a range of teeth, a dental arch (either maxillary or mandibular), both dental arches (maxillary and mandibular), a temporomandibular joint (TMJ), both TMJs, a whole dentition and TMJs. The scan request may further comprise object data representing at least one object related parameter (e.g. age, size, etc.) and/or imaging program data representing required imaging program (e.g. dose information including an indication of a dose to be used in the scan, resolution information including an indication of a resolution to be used in the scan, etc.). The scan request may also comprise patient identification data (for example, a patient name, a patient identification number, photograph, fingerprint, retinal scan, facial recognition, and/or other biometric data, etc.). The scan request may be received locally or remotely via a user interface part 140a, 140b. The user interface part 140a may for example be located at the same location as the dental X-ray imaging unit 102 is located, i.e. the scan request may be received locally. For example, the dental X-ray imaging unit 102 may comprise the user interface part 140a, e.g. a touch screen, as in the example dental X-ray imaging unit 102 of Figure 1. An operator of the dental X-ray imaging unit 102 may input the scan request via the user interface part 140a. Alternatively or in addition, the user interface part 140b may for example be located at another location than the location where the dental X-ray imaging unit 102 is located, i.e. the user interface part 140b may be located remotely from the dental X-ray imaging unit 102, and be communicatively coupled with the control system 106. In that case the scan request may be received remotely. According to an example, initial FOV data may be defined based on the received scan request. The initial FOV data may comprise initial position of the FOV and initial size of the FOV (i.e. initial dimensions of the FOV).

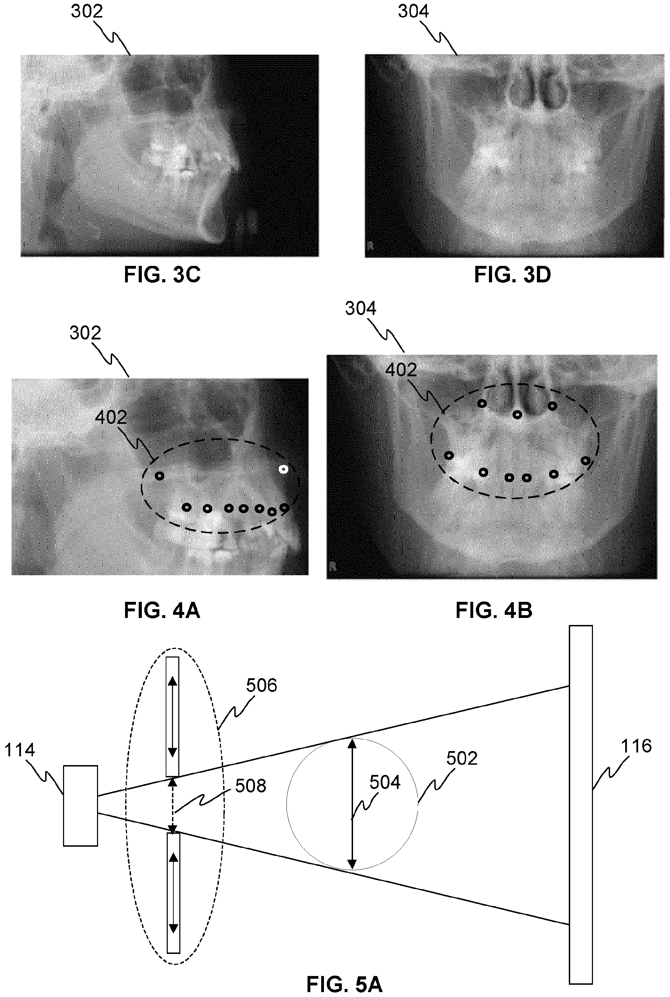

At a step 220 the control system 106 controls the parts of the dental X-ray imaging unit 102 to acquire two (non-parallel) X-ray scout images 302, 304 of the object 300, e.g. a first X-ray scout image 302 of the object 300 and a second scout image 304 of the object 300. The two X-ray scout images 302, 304 are two-dimensional (2D) X-ray projection images. Preferably, the two X-ray scout images 302, 304 are acquired orthogonal to each other, i.e. at 90 degrees angle. Most preferably, the two scout images comprise a lateral (LAT) view angle X-ray scout image 302, i.e. a LAT scout image, of the object 300, and a posterior-anterior (PA) view angle X-ray scout image 304, i.e. a PA scout image, of the object 300. Figure 3A illustrates schematically an example of the lateral imaging of the object 300 for acquiring the LAT scout image 302 of the object 300. Figure 3B illustrates schematically an example of the posterior-anterior imaging of the object 300 for acquiring the PA scout image 304 of the object 300. Figure 3C illustrates schematically an example of the LAT scout image 302 of the object 300 acquired by the lateral imaging. Figure 3D illustrates schematically an example of the PA scout image 304 of the object 300 acquired by the posterior-anterior imaging.

At as step 230, the control system 106 detects at least one target structure of the object 300 (e.g. at least one anatomical target structure of the object 300) from the two X-ray scout images 302, 304 associated with the at least one ROI indicated in the received scan request by using automated detection. The automated detection comprises applying at least one trained detection model 616 for the X-ray scout images 302, 304. In other words, at the step 230 the control system 106 detects the at least one target structure of the object 300 from the two X-ray scout images 302, 304 associated with the at least one ROI indicated in the received scan request by applying at least one trained detection model 616 for the X-ray scout images 302, 304. Yet in other words, at the step 230 the control system 106 detects the same at least one structure of the object 300 associated with the at least one ROI indicated in the received scan request from both X-ray scout images 302, 304 by applying at least one trained detection model 616 for the X-ray scout image 302, 304. The two X-ray scout images 302, 304 are used as the input data of the at least one trained detection model 616 and the at least one target structure of the object 300 associated with the at least one ROI is obtained as the output data of the at least one trained detection model 616. The detection of the at least one target structure of the object 300 means that the at least one target structure is localized i.e. its spatial location in each X-ray scout image 302, 304 is defined, and its identity (e.g. name of label) is defined. The expression "at least one target structure of the object 300 associated with the at least one ROI" means throughout this application that at least one target structure of the object 300 may be within an area of the at least one ROI (e.g. included in the at least one ROI) and/or be at least one anatomical structure of the object 300 (being outside the area of the at least one ROI) that may be used to detect at least one target structure of the object 300 within the area of the at least one ROI. In other words, the at least one target structure of the object 300 within the area of the ROI may be detected directly and/or indirectly.

The at least one trained detection model 616 may comprise at least one common trained detection model trained for both X-ray scout images 302, 304. In that case, the control system 106 may apply the at least one common trained detection model 616 for the first X-ray scout image 302 (e.g. the LAT scout image) to detect the at least one target structure of the object 300 associated with the at least one ROI indicated in the received scan request from the first X-ray scout image 302 and the same at least one common trained detection model 616 for the second X-ray scout image 304 (e.g. the PA scout image) to detect the at least one target structure of the object 300 associated with the at least one ROI indicated in the received scan request from the second X-ray scout image 304.

Alternatively, the at least one trained detection model may comprise different at least one detection model trained for each X-ray scout image 302, 304. As discussed above, the two X-ray scout images 302, 304 are non-parallel to each other. Thus, different at least one trained detection model 616 may be trained for different view angles. In other words, different at least one detection model 616 may be trained for the first X-ray scout image (e.g. the LAT scout image) 302 and different at least one detection model 616 may be trained for the second X-ray scout image (e.g. the PA scout image) 304. When the at least one trained detection model comprises different at least one detection model trained for each X-ray scout image 302, 304, the at least one detection model 616 trained for the first scout image 302 may for example be called as at least one trained first detection model and the at least one detection model trained for the second scout image 302 may for example be called as at least one trained second detection model 616. In this case, the control system 106 may apply the at least one trained first detection model 616 for the first X-ray scout image 302 (e.g. the LAT scout image) to detect the at least one target structure of the object 300 associated with the at least one ROI indicated in the received scan request from the first X-ray scout image 302 and the at least one trained second detection model 616 for the second X-ray scout image 304 (e.g. the PA scout image) to detect the at least one target structure of the object 300 associated with the at least one ROI indicated in the received scan request from the second X-ray scout image 304.

The at least one trained detection model 616 may depend on a region of the object 300 imaged in the X-ray scout images 302, 304. In other words, different trained detection models 616 may be trained for different regions of the object 300. For example, at least one trained detection model 616 may be trained for at least one of the following regions of the object 300: the region of maxillary teeth, the region of mandibular teeth, the region of both maxillary and mandibular teeth, and/or the region of TMJs, etc. At least one common trained detection model 616 for both scout images (e.g. LAT scout image and PA scout image) 302, 304 may be trained for different regions of the object 300. Similarly, at least one trained first detection model 616 for the first X-ray scout image 302 (e.g. the LAT scout image) may be trained for different regions of the object 300 and at least one trained second detection model 616 for the second X-ray scout image 304 (e.g. the PA scout image) may be trained for different regions of the object 300. Depending on the region 300 of the object 300 imaged in the X-ray scout images 302, 304, appropriate at least one trained detection model 616 for the X-ray scout images 302, 304 may be selected.

The at least one trained detection model 616 may be formed by using training data. The training of the at least one detection model 616 may preferably be supervised training, i.e. supervised learning, but also other training paradigms may be used, e.g. unsupervised learning, reinforcement learning, or hybrid paradigms comprising two or more training paradigms. In the supervised training, the training data comprises input training data and output training data. The input training data may for example comprise a plurality of X-ray scout images. The plurality of X-ray scout images comprised in the input training data may for example comprise previously collected X-ray scout images. The previously collected X-ray scout images may preferably comprise X-ray scout mages of patients. Alternatively or in addition, the previously collected X-ray scout images may comprise X-ray scout images of phantoms (e.g. dry skulls). Alternatively or in addition, the plurality of X-ray scout images comprised in the input training data may comprise simulated and/or artificial X-ray scout images. Data augmentation may also be used to produce further input training data. The output training data may for example comprise annotation data representing annotations of the anatomical structures, e.g. the positions and the identities of the anatomical structures. The output training data may be generated in a manual process, e.g. by a human expert, in a semi-automated process, or in a fully automated process. The at least one trained detection model 616 may be based on one or more machine learning (ML) methods or one or more artificial intelligence (Al) methods. In other words, the at least one trained detection model 616 may be a ML model or an AI model. For example, the at least one trained detection model 616 may be based on a tree-based machine learning (ML) methods, e.g. decisions trees, and especially regression trees. To leverage the power of a single tree, multiple trees may be combined (i.e. ensemble). Preferably, the at least one trained detection model 616 may be based on ensemble of regression trees ML method. Alternatively, the at least one trained detection model 616 may be based on convolutional neural networks (CNNs). The at least one trained detection model 616 may be stored into a memory part 608 of the control system 106. Although multiple trained detection models 616 may be used as discussed above, each trained detection model 616 is on the same ML method or AI method, e.g. the ensemble regression trees ML method or the CNNs.

The detecting of the at least one target structure of the object 300 from the two X-ray scout images 302, 304 may be based on landmark -based detection, bounding box -based detection, and/or object segmentation -based detection. A combination of the landmark -based detection and the bounding box -based detection may comprise bounding box detection of a region in each X-ray scout image 302, 304 followed by the landmark -based detection applied inside the detected regions. In the landmark -based detection at least one landmark associated with the at least one ROI is detected from each X-ray scout image 302, 304 by using the automated detection, i.e. by applying the at least one trained detection model 616 for the X-ray scout images 302, 304. In other words, each X-ray scout image 302, 304 may be used as the input data of the at least one trained detection model 616 and the at least one landmark 402 may be obtained as the output data of the at least one trained detection model 616. The expression "at least one landmark associated with the at least one ROI" means throughout this application that at least one landmark may be within the area of the at least one ROI (e.g. included in the at least one ROI) and/or be at least one landmark (being outside the area of the at least one ROI) that may be used to detect at least one landmark within the area of the at least one ROI. In other words, the at least one landmark within the area of the ROI may be detected directly and/or indirectly. Some non-limiting examples of the at least one landmark 402 may comprise mandibular teeth, maxillary teeth, the TMJs, anterior nasal spine (ANS), posterior nasal spine (PNS), and/or any other cranial structure. Some of the landmarks 402 may be directly related to the ROI and some of the landmarks 402 may be stable cranial structures that may be used as a proxy-information. For example, the ANS is located just above maxillary teeth roots, thus if the most superior position of maxillary roots needs to be estimated, the ANS may be used as the proxy-information on that. Figure 4A illustrates schematically a non-liming example of the landmarks 402 intended for detecting the maxillary teeth from the LAT scout image 302. Figure 4B illustrates schematically a non-liming example of the landmarks 402 intended for detecting the maxillary teeth from the PA scout image 304. In the examples of Figures 4A and 4B the small circles inside the dashed ellipse represent the landmarks 402. In the example of Figure 4A the used trained detection model 616 is trained for the LAT scout image 302 at the region of maxillary teeth. In the example of Figure 4B the used trained detection model 616 is trained for the PA scout image 304 at the region of maxillary teeth.

At a step 240, the control system 106 defines FOV data of the dental X-ray imaging unit 102 at least partially based on the at least one target structure detected at the step 230. The FOV data may comprise a FOV position and a FOV size (i.e. dimensions of the FOV). Preferably, at the step 240, the control system 106 defines the FOV data based on the at least one target structure detected at the step 230. Alternatively, if the initial FOV data is defined based on the scan request as discussed above, the control system 106 may define the FOV data partially based on the at least one target structure detected at the step 230 and partially based on the initial FOV data. For example, the FOV size (or at least one dimension of the FOV) may be defined based on the initial FOV data or the FOV position may for example be defined based on the initial FOV data. According to a non-limiting example, the FOV position may be defined based on the at least one target structure of the object detected at the step 230 and the FOV size may be defined based on the initial FOV data, or vice versa. The FOV position may for example be a center vertical axis of the FOV (i.e. the FOV center axis). If the at least one target structure comprises a single structure (e.g. a single tooth), the control system 106 may define the FOV center axis so that the FOV center axis goes (i.e. passes) through the center of the single structure. Alternatively, if the at least one target structure comprises multiple structures (e.g. multiple teeth), the control system 106 may define the FOV center axis so that the FOV center axis goes through the center of the multiple structures. The center of the multiple structures may for example be defined by using averaging (e.g. arithmetic mean, geometric mean, or weighted mean, etc.). The dimensions of the FOV may for example comprise a horizontal dimension of the FOV (e.g. a width of the FOV, i.e. a diameter of the FOV, or a radius of the FOV), and a vertical dimension of the FOV (e.g. a vertical height of the FOV). If the at least one target structure comprises a single structure, the control system 106 may for example estimate the horizontal dimension of the FOV based on the horizontal dimension of the structure. Alternatively, if the at least one target structure comprises multiple structures, the control system 106 may for example estimate the horizontal dimension of the FOV by defining for each structures the distance from the defined FOV center axis and the maximum of these distances may be used as the radius of the FOV. The width of the FOV, i.e. the diameter of the FOV, may be defined based on the radius of the FOV. The vertical dimension of the FOV may be defined based on a most superior point of the FOV and a most inferior point of the FOV. To define the most superior point of the FOV, the control system 106 may define the most superior point of the at least one target structure and assign the top of the FOV at the same level, i.e. the defined most superior point of the at least one target structure is assigned as the most superior point of the FOV. If the target at least one target structure comprises multiple structures, the most superior point of all of these multiple structures may be assigned as the most superior point of the FOV. To define the most inferior point of the FOV, the control system 106 may define the most inferior point of the at least one target structure and assign the bottom of the FOV at the same level, i.e. the defined most inferior point of the at least one target structure is assigned as the most inferior point of the FOV. If the at least one target structure comprises multiple structures, the most inferior point of all of these multiple structures may be assigned as the most inferior point of the FOV.

According to an example, a safety margin may be added to the horizontal dimension of the FOV and to the vertical dimension of the FOV. The size of the safety margin may for example be fixed or it may depend on the object 300, the ROI, and/or the imaging program data. The safety margin may be the same for the horizontal dimension of the FOV and for the vertical dimension of the FOV. Alternatively, different safety margins may be added to the horizontal dimension and to the vertical dimension of the FOV. According to an example, the object data and/or the imaging program data may be used in the defining of the FOV data, e.g. in the defining of the safety margin.

The FOV data may be defined in 3D imaging device coordinates (i.e. in the coordinates of the dental X-ray imaging unit 102) at the step 240. As the at least one target structure of the object 300 detected at the step 230 is presented in 2D image coordinates, the detected at least one target structure may be transformed into the 3D imaging device coordinates. The transformation may be performed by using imaging geometry data representing an imaging geometry of the dental X-ray imaging unit 102 respective to the two X-ray scout images 302, 304. The control system 106 may for example obtain the imaging geometry data when the two X-ray scout images are acquired, i.e. in connection with the acquirement of the two X-ray scout images, at the step 220. The control system 106 may transform the detected at least one target structure into the 3D imaging device coordinates by using the obtained imaging geometry data. Using the imaging geometry data, the location of at least one target structure detected from the X-ray scout images 302, 304 may be transformed into the 3D imaging device coordinates. For example, when the same target structure(s) is/are detected in the two X-ray scout images (e.g. in the LAT scout image 302 and in the PA scout image 304), the target structure(s) may be mapped to the 3D imaging device coordinates by using the obtained imaging geometry data. The transformation may for example be performed by applying an intersecting lines method. Next an example of applying the intersecting lines method for one target structure to transform the target structure into the 3D imaging device coordinates is described. However, the intersecting lines method may be applied similarly for each target structure to transform the target structure in question into the 3D imaging device coordinates. The location of the X-ray source in the imaging device coordinates may be defined from the imaging geometry data. Similarly, the location of the X-ray detector in the imaging device coordinates may be defined from the imaging geometry data. By combining the location of the X-ray detector and the detected target structure at the X-ray detector (i.e. at the X-ray scout image 302, 304), the location of the detected target structure at the X-ray scout image 302, 304 may be expressed in the imaging device coordinates. Thus, a line origin from the X-ray source into the location of the detected target structure at the X-ray detector may be formed in the imaging device coordinates. As there are two non-parallel X-ray scout images 302, 304, two non-parallel lines for the detected target structure may be formed. The location of the detected target structure in the imaging device coordinates may then be solved by applying the intersecting lines method. In addition to intersecting lines method there are many similar alternative methods, e.g. intersection of 2D plane and line, intersection of two lines at projected on a plane (e.g. horizontal plane), etc. Each of these alternative methods or any other known methods may be used to transform the at least one target structure into the 3D imaging device coordinates, when the imaging geometry data is available.

After the defining of the FOV data, at an optional step 250, the control system 106 may output via one or more user interface devices, e.g. the user interface part 140a, the defined FOV data superimposed on top of the X-ray scout images 302, 304 to the operator of the dental X-ray imaging unit 102 for a review and approval. This enables that the operator may review the correctness of the defined FOV data. If the operator is satisfied with the defined FOV data, the operator may approve the defined FOV data. Alternatively, if the operator is not satisfied with the defined FOV data, the operator may manually adjust the FOV position and/or at least one dimension of the FOV via the user interface part 140a before approving the FOV data.

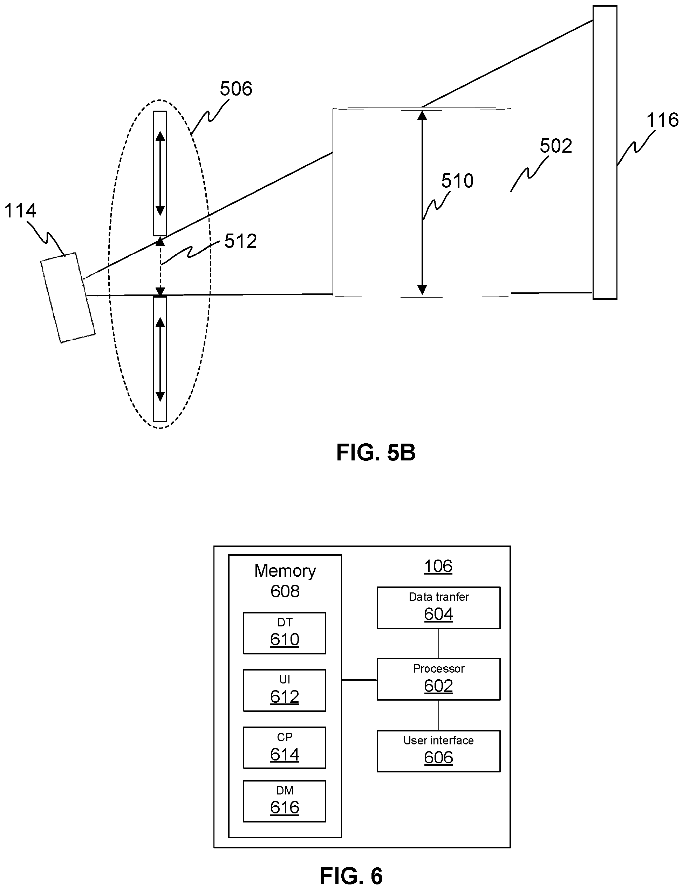

At a step 260, the control system 106 controls the parts of the dental X-ray imaging unit 102 to implement the defined FOV data for the CT scan of the object 300 in order to acquire dental CT X-ray image data of the object 300. In case of a symmetrical CT imaging, the control system 106 controls the gantry part 112 of the dental X-ray imaging unit 102 to move into a starting position of the CT scan, wherein the rotation axis is aligned with the central axis of the FOV defined at the step 240. The rotation axis may be a mechanical rotation axis of the gantry part 112 or a virtual rotation axis. The virtual rotation axis may be obtained, for example, by moving the mechanical rotation axis of the gantry part 112 along a circular path, whereupon the virtual rotation axis may be formed in the centre of said circular path. In case of an off-set CT-imaging, the starting position of the CT scan may be off-set from the central axis of the FOV defined at the step 240 by a known amount in a known direction, i.e. by a known off-set vector. The control system 106 may adjust the horizontal dimension 504 of the FOV 502 to correspond to the defined horizontal dimension of the FOV by means of the collimator 506 of the X-ray source part 114. The vertical dimension 510 of the FOV 502 may be adjusted to correspond to the defined vertical dimension of the FOV by means of the collimator 506. For example, vertical dimension 510 of the FOV 502 may be adjusted by controlling the collimator 506 of the X-ray source part 114 so that the most superior point of the FOV 502 corresponds to the defined most superior point of the FOV and/or that the most inferior point of the FOV 502 corresponds to the defined most inferior point of the FOV. The collimator 506 of the X-ray source part 114 may comprise an adjustable slit. The size of the slit of the collimator 506 may be adjusted in the horizontal direction to adjust the horizontal dimension 504 of the FOV 502 and in the vertical direction to adjust the vertical dimension 510 of the FOV 502. Figure 5A illustrates schematically an example of adjusting the horizontal dimension 504 of the FOV 502. The horizontal dimension 504 in this example is the width of the FOV 502, i.e. the diameter of the FOV 502. The diameter 504 of the of the FOV 502 may be adjusted by adjusting the size of the slit of the collimator 506 in the horizontal direction. The size of the slit of the collimator 506 in the horizontal direction, i.e. the width of the slit, is illustrated in Figure 5A with the dashed arrow 508. The larger the slit of the collimator 506 is in the horizontal direction the larger the diameter 504 of the FOV 502 is. In other words, by closing the slit of the collimator 506 in the horizontal direction, the diameter 504 of the FOV 502 may be adjusted smaller and by opening the slit of the collimator 506 in the horizontal direction, the diameter 504 of the FOV 502 may be adjusted larger. Figure 5B illustrates schematically an example of adjusting the vertical dimension 510 of the FOV 502. The vertical dimension 510 in this example is the height of the FOV 502. The height 510 of the of the FOV 502 may be adjusted by adjusting the size of the slit of the collimator 506 in the vertical direction. The size of the slit of the collimator 506 in the vertical direction, i.e. the height of the slit, is illustrated in Figure 5B with the dashed arrow 512. The larger the slit of the collimator 506 is in the vertical direction, the larger the height 510 of the FOV 502 is. In other words, by closing the slit of the collimator 506 in the vertical direction, the height 510 of the FOV 502 may be adjusted smaller and by opening the slit of the collimator 506 in the vertical direction, the height 510 of the FOV 502 may be adjusted larger.

At a step 270, after the rotation axis is moved into the starting position of the CT scan and the horizontal dimension and the vertical dimension of the FOV are adjusted at the step 260, the control system 106 controls the parts of the dental X-ray imaging unit 102 to scan the object (300) in order to acquire the dental CT X-ray image data of the object 300. The controlling at the step 270 may comprise controlling the gantry part 112 to rotate according to a motion path of the CT scan around the rotation axis in order to acquire the dental CT X-ray image data of the object 300. The motion path may for example be a circular path or a non-circular path, e.g. an elliptic path, around the rotation axis. The circular path or the non-circular path may be full rotation or partial rotation around the rotation axis. The non-circular rotation path may be produced, for example, by moving the gantry part 112 along a motion path deviating from the circular path, for example an elliptic path. Other techniques or alignments for the rotation axis may also be used as will be recognized by a person or ordinary skill in the art.

Figure 6 illustrates a schematic example of the control system 106 of the dental X-ray imaging system 100. The control system 106 may comprise a processor part 602, a data transfer part 604, a user interface part 606, and a memory part 608. The processor part 602 is configured to perform user and/or computer program (software) initiated instructions, and to process data. The processor part 602 may comprise at least one processor. The memory part 608 is configured to store and maintain data. The data may be instructions, computer programs, the at least one trained detection model 616, and any data files. The memory part 608 may comprise at least one memory. The memory part 608 may further comprise at least a data transfer application 610 in order to control the data transfer part 604, a user interface application 612 in order to control the III part 606, and a computer program (code) 614 in order to control the operations of the control system 106. The memory part 608 and the computer program 614, together with the processor part 602, may cause the control system 106 at least to implement one or more method steps and/or operations of the control system 106 as described above. The data transfer part 604 may be configured to send control commands other units, e.g. the dental X-ray imaging unit 102. In addition, the data transfer part 604 may receive data from other units, e.g. the dental X-ray imaging unit 102, the user interface part 140b, a database(s) and/or any other external units. The user interface (UI) part 606 may be configured to input control commands, to receive information and/or instructions, and to display information. The UI part 606 may comprise at least a display, a screen, a touchscreen, at least one function key, a keyboard, a wired or wireless remote controller, or any other user input and/or output device. The computer program 614 may be a computer program product that may be comprised in a tangible, non-volatile (non-transitory) computer-readable medium bearing the computer program code 614 embodied therein for use with a computer, e.g. the control system 106.

The CT dental X-ray imaging method described above enables reducing the imaging time, because manual FOV positioning by the operator of the dental X-ray imaging unit 102 is not needed. Furthermore, the FOV positioning does not depend on the anatomy knowledge and skills by the operator of the dental X-ray imaging unit 102. Therefore, the CT dental X-ray imaging method described above improves the imaging accuracy. Although the FOV positioning does not depend on the anatomy knowledge and skills by the operator, the CT dental X-ray imaging method described above provides support for the skills of the operator.

The specific examples provided in the description given above should not be construed as limiting the applicability and/or the interpretation of the appended claims. Lists and groups of examples provided in the description given above are not exhaustive unless otherwise explicitly stated.

## Claims

1. A dental X-ray imaging system (100) for a computed tomography (CT) dental X-ray imaging of an object (300), the system (100) comprising:
a dental X-ray imaging unit (102) comprising:
an X-ray source part (114) for emitting X-rays,
an X-ray imaging detector part (116) for receiving the X-rays from the source part (114), and
a gantry part (112) comprising the X-ray source part (114) and the X-ray imaging detector part (116); and
a control system (106) configured to:
receive scan request comprising an indication of at least one region of interest (ROI);
control the parts of the dental X-ray imaging unit (102) to acquire two X-ray scout images (302, 304) of the object (300);
detect at least one target structure of the object (300) from the two X-ray scout images (302, 304) associated with the at least one ROI by applying at least one trained detection model (616) for the X-ray scout images (302, 304);
define field of view (FOV) data of the dental X-ray imaging unit (102) at least partially based on the detected at least one target structure; and
control the parts of the dental X-ray imaging unit (102) to implement the defined FOV data for a CT scan the object (300) in order to acquire dental CT X-ray image data of the object (300).

2. The dental X-ray imaging system (100) according to claim 1, wherein the FOV data comprises a FOV position and a FOV size.

3. The dental X-ray imaging system (100) according to any of the preceding claims, wherein the two X-ray scout images (302, 304) of the object comprise a lateral (LAT) view angle X-ray scout image (302) and a posterior-anterior (PA) view angle X-ray scout image (304).

4. The dental X-ray imaging system (100) according to any of the preceding claims, wherein the control system (106) is further configured to:
obtain imaging geometry data representing an imaging geometry of the dental X-ray imaging unit (102) respective to the two X-ray scout images (302, 304); and
transform the detected at least one target structure into 3D device coordinates by using the obtained imaging geometry data.

5. The dental X-ray imaging system (100) according to any of the preceding claims, wherein the scan request further comprises object data representing at least one object related parameter and/or imaging program data representing required imaging program.

6. The dental X-ray imaging system (100) according to claim 5, wherein the object data and/or the imaging program data is used in the defining of the FOV data.

7. The dental X-ray imaging system (100) according to any of the preceding claims, wherein the at least one trained detection model (616) is a machine learning (ML) model or an artificial intelligence (AI) model.

8. The dental X-ray imaging system (100) according to any of the preceding claims, wherein the at least one trained detection model (616) comprises at least one common trained detection model trained for both X-ray scout images (302, 304).

9. The dental X-ray imaging system (100) according to any of claims 1 to 7, wherein the at least one trained detection model (616) comprises different at least one trained detection model trained for each X-ray scout image (302, 304).

10. The dental X-ray imaging system (100) according to any of the preceding claims, wherein the at least trained detection model (616) depends on a region of the object (300) imaged in the X-ray scout images (302, 304).

11. The dental X-ray imaging system (100) according to any of the preceding claims, wherein the detecting the at least one target structure of the object (300) from the two X-ray scout images (302, 304) is based on landmark -based detection, bounding box -based detection, and/or object segmentation -based detection.

12. The dental X-ray imaging system (100) according to claim 11, wherein in the landmark -based detection at least one landmark (402) associated with the at least one ROI is detected from the two X-ray scout images (302, 304) by applying the at least one trained detection model (616) for the X-ray scout images (302, 304).

13. A method for a computed tomography (CT) dental X-ray imaging, the method is performed by an X-ray dental imaging system (100) according to any of the preceding claims, wherein the method comprises:
receiving (210) scan request comprising an indication of at least one region of interest (ROI);
controlling (220) the parts of the dental X-ray imaging unit (102) to acquire two X-ray scout images (302, 304) of the object (300);
detecting (230) at least one target structure of the object (300) from the two scout images (302, 304) associated with the at least one ROI by applying at least one trained detection model (616) for the X-ray scout images (302, 304);
defining (240) field of view (FOV) data of the dental X-ray imaging unit (102) at least partially based on the detected at least one target structure; and
controlling (260) the parts of the dental X-ray imaging unit (102) to implement the defined FOV data for a CT scan of the object (300) in order to acquire CT dental X-ray image data of the object (300).

14. A computer program (614) comprising instructions which, when the program (614) is executed by an X-ray dental imaging system (100) according to any of claims 1 to 12, cause the X-ray dental imaging system (100) to carry out the method according to claim 13.

15. A tangible non-volatile computer-readable medium comprising instructions which, when executed by an X-ray dental imaging system (100) according to any of claims 1 to 12, cause the X-ray dental imaging system (100) to carry out the method according to claim 14.
